(19) Europäisches Patentamt
European Patent Office
Office européen des brevets

(11) **EP 2 590 557 B1**

(12) **EUROPEAN PATENT SPECIFICATION**

(45) Date of publication and mention
of the grant of the patent:
**10.08.2016 Bulletin 2016/32**

(21) Application number: **11758274.2**

(22) Date of filing: **30.06.2011**

(51) Int Cl.:
**A61B 5/11** (2006.01)    **A61B 5/22** (2006.01)

(86) International application number:
**PCT/SI2011/000034**

(87) International publication number:
**WO 2012/005701 (12.01.2012 Gazette 2012/02)**

(54) **DEVICE FOR NON-INVASIVE AND SELECTIVE DETERMINATION OF BIOMECHANICAL, CONTRACTILE AND VISCOELASTIC PROPERTIES OF SURFACE SKELETAL MUSCLES**

VORRICHTUNG ZUR NICHTINVASIVEN UND SELEKTIVEN BESTIMMUNG BIOMECHANISCHER, KONTRAKTILER UND VISKOELASTISCHER EIGENSCHAFTEN VON OBERFLÄCHENSKELETTMUSKELN

DISPOSITIF DE DÉTERMINATION NON INVASIVE ET SÉLECTIVE DES PROPRIÉTÉS BIOMÉCANIQUES, CONTRACTILES ET VISCOÉLASTIQUES DE MUSCLES SQUELETTIQUES SUPERFICIELS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.07.2010 SI 201000203**

(43) Date of publication of application:
**15.05.2013 Bulletin 2013/20**

(73) Proprietor: **TMG-BMC, d.o.o.**
**1000 Ljubljana (SI)**

(72) Inventors:
• **TOMAZIC, Saso**
**1000 Ljubljana (SI)**
• **DORDEVIC, Srdan**
**1000 Ljubljana (SI)**

(74) Representative: **Pipan, Marjan**
**Kotnikova 5**
**1000 Ljubljana (SI)**

(56) References cited:
**WO-A1-02/074167      WO-A1-2008/105590**
**DE-A1-102008 038 234**

• **RICHARD H BROWN: 'The Piezo Solution for Vital Signs Monitoring', [Online] 11 March 2008, XP055252948 Retrieved from the Internet: <URL:http://www.mdtmag.com/article/2008/03/piezo-solution-vital-signs-monitoring> [retrieved on 2016-02-24]**
• **Shunji Moromugi ET AL: "Muscle stiffness sensor to control an assistance device for the disabled", Artificial Life and Robotics, vol. 8, no. 1, 1 September 2004 (2004-09-01), pages 42-45, XP055252732, Tokyo ISSN: 1433-5298, DOI: 10.1007/s10015-004-0286-8**

## Description

A measuring method and device have been developed to enable the determination of the biomechanical, contractile and viscoelastic properties (in the following text referred to as *BCVP*) of all surface skeletal muscles, muscle parts, tendons and ligaments (in following text referred to as *the subject of measurement*), which can be performed *in situ* (examine the phenomenon exactly in place where it occurs) in a completely non-invasive way. Furthermore, the presented method and device enable selective measurements, meaning that the activity of individual muscle segments can be distinguished. According to the proposed method and by using the proposed device, BCVP determination is achieved by measuring muscle force at the skin surface above the subject of measurement. Placement of the measuring device at the skin surface and measurement performance does not cause any pain or discomfort to the individual being measured. The invention presented falls under the biomechanics field of international patent classification.

The presented method and device successfully enable BCVP determination of all mentioned subjects *in situ* by completely non-invasive and selective measurement.

Skeletal muscles constitute the largest organ in the human body. Furthermore, they are the largest consumer of energy and enable efficient movement at varying intensity and duration along different patterns of motion. Various muscle fibres increase the range of demands that skeletal muscle can accommodate.

A healthy muscular system is associated with healthy cardiovascular, pulmonary, immune and endocrine systems (S. Nielsen, B.K. Pedersen, Skeletal muscle as an immunogenic organ, Curr Opin Pharmacol, 2008 Jun, 8(3), p346-51.*; L.R. Leiber, Skeletal Muscle Structure, Function and Plasticity: The Physiological Basis of Rehabilitation, Lippincott Williams and Wilkins, 2nd Revised edition (2002*).; B.K. Pedersen, T.C.A. Akerström, A. R. Nielsen, C.P. Fischer, Role of myokines in exercise and metabolism, Appl Physiol, 2007 Sep, 103(3), p1093-8.). Conversely, disorders of the neuromuscular system have dramatic effects on daily activities and independence (L.R. Leiber, Skeletal Muscle Structure, Function and Plasticity: The Physiological Basis of Rehabilitation, Lippincott Williams and Wilkins, 2nd Revised edition (2002*)). The beneficial health effects of exercise (through muscular activity) play important roles in the protection against diseases associated with low-grade inflammation, such as cardiovascular diseases, type-2 diabetes, symptoms related to the metabolic syndrome and cancer (B.K. Pedersen, C.P. Fischer, Beneficial health effects of exercise--the role of IL-6 as a myokine, Trends Pharmacol Sci., 2007 Apr, 28(4), p152-6.). Also, muscle activity and resistance training can initiate some anti-aging effects (R. Koopman, L.J.C. van Loon, Aging, exercise, and muscle protein metabolism, J Appl Physiol, 2009 Jun, 106(6), p2040-8.). Furthermore, resistance exercise training can reduce markers of oxidative stress and increase anti-oxidant enzyme activity in older adults (G. Parise, S.M. Phillips, J.J. Kaczor, M.A. Tamopolsky, Antioxidant enzyme activity is up-regulated after unilateral resistance exercise training in older adults, Free Radic Biol Med. 2005 Jul 15, 39(2), p289-95.; G. Parise, A.N. Brose, M.A. Tarnopolsky, Resistance exercise training decreases oxidative damage to DNA and increases cytochrome oxidase activity in older adults, Exp Gerontol. 2005 Mar, 40(3), p173-80.).

Understanding such universal phenomena as movement or strength requires an understanding of the complete neuromuscular system (L.R. Leiber, Skeletal Muscle Structure, Function and Plasticity: The Physiological Basis of Rehabilitation, Lippincott Williams and Wilkins, 2nd Revised edition (2002*)). The current methods used to determine the biomechanical and contractile properties of skeletal muscles are wide-ranging; each method has its advantages and disadvantages but predominantly measures only a single component, which only defines how a muscle works under particular test conditions. Furthermore, the majority of tests are performed in a laboratory (B.K. Higginson, Methods of running gait analysis, Curr Sports Med Rep, 2009 May-Jun, 8(3), p136-41.), are constrained and often involve invasive methods (such as needle electromyogram, biopsy and maximum force measurement).

The direct determination of biomechanical properties in human skeletal muscles with the estimation of muscle-fibre-type percentage is usually assessed by applying histochemical and immunocytochemical techniques. Both techniques are based on myofibrillar adenosinetriphosphatase (M-ATP-aze) activity and myosin heavy chain isoform identification. Both techniques are applied to samples obtained by muscle biopsy and are therefore considered invasive and not suitable for routine application.

Because of the invasive character of direct methods, muscle function and property measurement is usually performed according to some indirect measurement method. Indirect methods allow for the estimation of the strength of skeletal muscle or a group of muscles.

The biomechanical properties of human skeletal muscles have usually been detected indirectly by measuring muscle force or torque about a specific joint. Clarkson and Gilewich (H. Clarkson, G. Gilewich, Musculoskeletal assessment: joint range of motion and manual muscle strength, Williams and Wilkins, 1989.) have defined 'muscle strength' as the maximum amount of force or tension (exerted by a group of muscles) or muscle force (exerted by a single muscle) in performing maximum voluntary contraction (MVC) under specific conditions (type of contraction, joint angle). To perform such (direct) measurements properly, it would be necessary to attach the measuring mechanism to/in a muscle tendon. Although such measurements have been accomplished, they have not been applied in clinical or sports practice.

The methods used to measure muscle force or muscle torque still represent a technical problem, which has, to some extent, been solved by using devices, such as the manual dynamometer, the wire-tensiometer or the isokinetic dynamometer, to estimate the mechanical properties of skeletal muscles. Unfortunately, none of the measuring methods features a dominant advantage that would make it generally applicable.

The manual dynamometer is based on a hydraulic, spring or strain-gauge system and is held by the measurer. The individual performs a pull at MVC against the measurer. The measurement is conducted under isometric or eccentric conditions. The devices is portable, relatively inexpensive- and within commercial reach. Its main disadvantage is non-selectivity and limb fixation, which makes the measuring process subjective (measurer-dependent). Moreover, no data regarding the dynamic properties of muscles can be obtained (very important for persons with diminished motor capabilities).

When using the wire-tensiometer measuring device, isometric muscle force is measured by one end of a wire attached to a fixed holder; the other end is attached to the limb of the individual. This device is mainly used for research purposes (research laboratories), is inexpensive and provides reliable measuring results in healthy individuals. Its disadvantages are non-selectivity and insufficient sensitivity when muscle force is very low. Again, no data regarding the dynamic properties of muscles can be obtained.

The isokinetic dynamometer is used to measure greater muscle forces at joint rotation with constant speed. Movements are performed either under isokinetic-concentric or isokinetic-eccentric conditions. In the clinical environment, this method is used for therapeutic as well as exercise purposes. The device itself is quite big and expensive. Calibration with external weights is required. To perform the measurements correctly, the device axes must fit joint axes, which is almost impossible to achieve. Moreover, the device cannot be used if the measuring individual is very tall or short. Measurements performed with this device are non-selective, as it is generally impossible to measure the force or torque exerted by a particular muscle; thus, only active muscle are measured. For knee extensions, the measured force refers to the composite activity of the *vastus lateralis, vastus medialis, rectus femoris* and *gastrocnemius.* "Most isokinetic testing machines are good enough to keep the angular velocity constant during testing. However, detailed real-time studies of quadriceps muscles have shown that muscle fibre velocity and moment arm are basically never constant during the test. This means that it is extremely problematic to interpret isokinetic data in terms of the muscles generating the torque. The results from these types of studies are often vastly overstated and over-interpreted" *(B.K. Higginson, Methods of running gait analysis, Curr Sports Med Rep, 2009 May-Jun, 8(3), p136-41.).* It is difficult to rigorously interpret torque-velocity data collected via isokinetic measurement because a number of factors are typically unknown. These include the following:

1. Muscle physiological cross-sectional area (PCSA) (T. Fukunaga, R.R. Roy, F.G. Shellock, J.A. Hodgson, M.K. Day, P.L. Lee, H. Kwong-Fu, V.R. Edgerton, Physiological cross-sectional area of human leg muscles based on magnetic resonance imaging, J Orthop Res, 1992 Nov, 10(6), p928-34.),

2. The fraction of the muscle's PCSA that is activated (R.M. Enoka, A.J. Fuglevand, Neuromuscular basis of the maximum voluntary force capacity of muscle, M. D. Grabiner (Ed.) Current Issues in Biomechanics, 1993, p215-235.)

3. Absolute moment arm as a function of joint angle and velocity (M. Ito, H. Akima, T. Fukunaga, In vivo moment arm determination using B-mode ultrasonography, J Biomech, 2000 Feb, 33(2), p215-8.)

4. Muscle fiber length as a function of joint angle and velocity (Y. Ichinose, Y. Kawakami, M. Ito, H. Kanehisa, T. Fukunaga, In vivo estimation of contraction velocity of human vastus lateralis muscle during "isokinetic" action, J Appl Physiol, 2000 Mar, 88(3), p851-6.)

5. Tendon length as a function of joint angle and velocity (M. Ito, H. Akima, T. Fukunaga, In vivo moment arm determination using B-mode ultrasonography, J Biomech, 2000 Feb, 33(2), p215-8.)

6. Inertial properties of the joint (R.L. Lieber, Isokinetic Dynamometers Used in Physical Assessment, Skeletal Muscle Structure, Function, and Plasticity, 3rd ed., Lipincott Williams & Wilkins, 2010, p119-124.)

These factors are considered independently as the limitations of isokinetic dynamometers *(B.K. Higginson, Methods of running gait analysis, Curr Sports Med Rep, 2009 May-Jun, 8(3), p136-41.).* Different technologies can diminish some of these limitations in musculoskeletal diagnostics.

Another large group of devices used to measure the biomechanical properties of skeletal muscles is based on detecting body-movement velocity or movements of specific parts of the body. The velocity parameter is present in individuals' everyday activity, can be controlled and regulated during sports activity and can vary in individuals with neuromuscular system malfunction. Movement velocity is determined by muscle contraction velocity, which is related to the speed of generating inner muscle tension and altogether depends on muscle fibre composition. Unfortunately, many other factors also impact movement velocity: mass of body segments, muscle length, physical condition, body and outer temperature, inner friction, gravity and other physiological factors. As muscle fibre composition is not the only factor that impacts movement velocity, the relevance of the results obtained in such a way can be disputable.

Traditional measurement devices, such as motion-capture systems, force plates and accelerometers, are adequate methods of gait analysis but have several limitations, such as high cost and lack of portability (B.K. Higginson, Methods of running gait analysis, Curr Sports Med Rep, 2009 May-Jun, 8(3), p136-41.).

Other established methods used to measure muscle properties and musculoskeletal disorders also have some limitations. Scientists and medical doctors are often interested in muscle function. They observe force development during contraction by either indirect (mainly muscle torque) or direct measurements; in most cases, electrical activity is monitored by an electromyogram (EMG).

The EMG provides only an 'intererferogram' that represents the summated electrical activation pattern of the muscle near the electrode. Since muscle force is highly dependent on length (due to the length-tension property) and velocity (due to the force-velocity property), electrical activity alone cannot possibly provide an accurate measurement of muscle force. In addition, because the EMG summates electrical activity in a way that does not uniquely represent all of the motor units activated, EMG measurements that are used to infer force are highly suspect (B.K. Higginson, Methods of running gait analysis, Curr Sports Med Rep, 2009 May-Jun, 8(3), p136-41.; L. Gerilovsky, P. Tsvetinov, G. Trenkova, Peripheral effects on .the amplitude of monopolar and bipolar H-reflex potentials from the soleus muscle, Exp Brain Res, 1989, 76(1), p173-81.; B. Bigland-Ritchie, EMG/force relations and fatigue of human voluntary contractions, Exerc Sport Sci Rev, 1981, 9, p75-117.; A.L. Hof, J. Van den Berg, EMG to force processing I: An electrical analogue of the Hill muscle model, J Biomech, 1981, 14(11), p747-58.; A.L. Hof, J. Van den Berg, EMG to force processing II: Estimation of parameters of the Hill muscle model for the human triceps surae by means of a calfergometer, J Biomech, 1981, 14(11), p759-70.; A.L. Hof, J. Van den Berg, EMG to force processing III: Estimation of model parameters for the human triceps surae muscle and assessment of the accuracy by means of a torque plate, J Biomech, 1981, 14(11), p771-85.; A.L. Hof, J. Van den Berg, EMG to force processing IV: Eccentric-concentric contractions on a spring-flywheel set up, J Biomech, 1981, 14(11), p787-92.; H.S. Milner-Brown, R.B. Stein, The relation between the surface electromyogram and muscular force, J Physiol, 1975 Apr, 246(3), p549-69.).

One of the limitations of the interference EMG is the variability in recording when the same task is performed by different individuals or by the same individual on different days. The two principal reasons for this variability are that the recording conditions change each time the electrodes are attached and the recording volume of the electrodes is usually less than the muscle mass involved in the task (R.M. Enoka, Electromyography, Neuromechanics of human movement, Champaign, USA: Human Kinefics, 2002, p46.55.).

The mechanomyography (MMG) method is a relatively new, noninvasive technique that records and quantifies the low-frequency lateral oscillations produced by active skeletal muscle fibres (T.W. Beck, T.J. Housh, J.T. Cramer, et al., Mechanomyographic amplitude and frequency responses during dynamic muscle actions: a comprehensive review, BioMed Eng OnLine, 2005, 4(1), p67.; T. W. Beck, T J. Housh, G.O. Johnson, et al., Mechanomyographic and electromyographic amplitude and frequency responses during fatiguing isokinetic muscle actions of the biceps brachii, Electromyogr Clin Neurophysiol, 2004, 44(7), p431-444.; C. Orizio, Sound myogram and EMG cross-spectrum during exhausting isometric contractions in humans, J Electromyogr Kinesiol, 1992, 2(3), p141-149.; C. Orizio, Muscle sound: bases for the introduction of a mechanomyographic signal in muscle studies, Crit Rev Biomed Eng, 1993, 21(3), p201-243., G. Gordon, A.H.S. Holboum, The sounds from single motor units in a contracting muscle, J Physiol, 1948, 107(4), p456-464.). It has been suggested that the lateral oscillations are the result of the following:

(a) gross lateral movement of the muscle as it moves towards or away from its line of pull during contraction or relaxation,
(b) smaller subsequent lateral oscillations generated at the resonant frequency of the muscle, and
(c) dimensional changes in the active muscle fibres (T.W. Beck, T.J. Housh, J.T. Cramer, et al., Mechanomyographic amplitude and frequency responses during dynamic muscle actions: a comprehensive review, BioMed Eng OnLine, 2005, 4(1), p67.; C. Orizio, Sound myogram and EMG cross-spectrum during exhausting isometric contractions in humans, J Electromyogr Kinesiol, 1992, 2(3), p141-149.). Lateral muscle fibre oscillations (quantified at the skin as MMG) reflect the intrinsic mechanical properties of motor unit activity.

It is important for future research to continue examining MMG amplitude and frequency responses during both dynamic and isometric muscle contraction in an effort to fully assess the potential uses/applications of MMG (T.W. Beck, T.J. Housh, J.T. Cramer, J. P. Weir, G.O. Johnson, J.W. Cobum, M.H. Malek, M. Mielke, Mechanomyographic amplitude and frequency responses during dynamic muscle actions: a comprehensive review, Biomed Eng Online, 2005 Dec 19, 4(1), p67.).

The TMG measuring technique (R. Dahmane, V. Valenčič, N.Knez, I. Eržen, Evaluation of the ability to make non-invasive estimation of muscle contractile properties on the basis of the muscle belly response, Medical and Biological Engineering and Computing, 2006, 39(1), p51-55.) was devised to avoid the invasive or indirect measurement of the biomechanical, dynamic and contractile properties of human skeletal muscles. This technique is based on the selective tensiomyographic measurement of muscle belly displacement, where muscle belly displacement is proportional to muscle force. Tensiomyographic data provides the biomechanical and contractile properties of a measured skeletal muscle or muscle group. Apart from non-invasiveness, selectivity and simple application, tensiomyographic devices also offer high sensitivity, which enables the detection of weak contractions. Such contractions are produced by muscles weakened by neuromuscular diseases, denervation or muscle atrophy (inactivity). This measuring technique and apparatus can be applied to measure the properties of human skeletal muscles while the measured individual is still and not performing any voluntary body movement. Furthermore, despite the non-invasive character of this measurement method, the measurement itself is not completely painless or unpleasant for the measuring individual.

The measuring method and device involved in this disclosure enable the determination of the BCVP (biomechanical, contractile and viscoelastic properties) of subjects (skeletal muscles, muscle parts, tendons and ligaments), which is an important element regarding the health and medical fields as well as professional sports, physiotherapy and ergonomy. Skeletal muscles enable the execution of various human movements and physical activities. Successful determination of the BCVP of all skeletal muscles and associated connective tissue ensures a complete understanding of the muscular and skeletal systems and consistent functional diagnostics.

In a kinematic chain, a group of body segments that are connected by joints operate together to provide a diverse range of movement. Distinguishing the activity of individual muscle segments in such a kinematic chain is referred to as selectivity in the measurement of the properties of skeletal muscle and associated connective tissue. When performing measurements to determine skeletal muscle BCVP, it is important to distinguish single-muscle contractions from group-muscle contractions. It is also important to distinguish activity and determine the BCVP of a single part of a skeletal muscle, for example the lateral and medial part of the gastrocnemius muscle. When analysing the functions of particular muscles active in such a kinematic chain, distinctions between single-muscle contractions can enable, for example, muscle injury diagnostics.

It is also important that measurements regarding BCVP determination are performed in a non-invasive way *in situ.* As skeletal muscles are considered an integrated system of the human body, interfering with them in an invasive way often contradicts the aim of the data-collecting procedure (e.g., it is senseless to conduct a biopsy on athletes - the process of extracting a muscle-fibre sample would only cause pain and demand minimum sports activity to allow the wound to heal properly). Moreover, the needle biopsy required for this procedure is prohibited in many European states (ethical code).

It is convenient to perform BCVP measurements regarding all skeletal muscles (e.g., *biceps brachii, brachioradialis, soleus* or *trapezius*), muscle parts, tendons and ligaments in the same way and to use the same measuring equipment. Skeletal muscles come in different shapes and sizes. By using the same measuring equipment to determine the BCVP of different skeletal muscles, measurers can perform measurements more easily and faster than when using different measuring equipment for different skeletal muscles.

Skeletal muscles are responsible for body movement during different physical activities. When performing physical activity (sequences of volatile contraction and retractions), the muscle activation pattern and recruitment order can change. During their activity, muscles can operate for different purposes and under different conditions. For this reason, performing measurements on moving individuals and detecting these muscle activity changes allows for more comprehensive BCVP determination than when measurements are performed only on still individuals.

Therefore, performing non-invasive *in situ* BCVP determination presents a significant step towards the understanding of skeletal muscle biomechanical and contractile properties and, consequently, towards better functional diagnostics.

The innovation behind the measuring method and device described in this document relies on force measurement performed to determine BCVP. During skeletal muscle activity, the muscle tension generated by muscle fibres changes. Skeletal muscles are able to produce varying levels of contractile force, which induce different levels of tension. The measurement of tension changes using this new device is achieved by measuring the force on the individual's skin above the muscle under study. Using this innovative measurement device enables *in situ* BCVP determination in a completely non-invasive way.

The present invention is defined in the appended claims. Document DE 10 2008 038 234 A1 discloses the features of the preamble of claim 1.

The essential parts that comprise the measuring device are a sensor with a sensor tip that enables force detection, a microprocessor and a supporting part that provides for the proper positioning of the measuring device. According to the measuring method, the device is pressed to the individual's skin above the skeletal muscle or muscle part that is of interest. The device is constructed in such a way that its pressing upon the individual's skin causes the sensor tip to strain the skin surface and the intermediate layer between the skin surface and the skeletal muscle, ultimately putting pressure on the subject of measurement (muscle, muscle part, tendon or ligament). The sensor must be suitably shaped so that it can be pressed into the individual's skin at the appropriate position in a non-invasive way. Any suitable force or pressure meter can be included in the device to measure the force detected at the sensor tip. The supporting part, along with a specially designed attachment made of an adhesive plaster, provides for the suitable attachment and fixation of the device on the surface of the measuring individual's skin.

Through this innovative measuring method, the measurement device can be attached to the individual's skeletal muscle in such a way that it remains evenly attached even if the measuring individual performs some movement or activity during the measurement procedure. The specific design of the innovative measuring device and all of its components do not limit the measuring individual's movement. Therefore, the measuring method and device can be evenly applied for the BCVP determination of still and moving individuals who may be involved in some activity.

Moreover, the specific design of the innovative measuring device and all of its components make the device and measuring method applicable for all skeletal muscles, including large gluteal muscles and small finger muscles.

The method and device can also be applied to determine the BCVP of other mammals and all animals with similar musculoskeletal structure.

Using a sample case, the invention is explained in detail through the following figures.

**Figure 1**:   Measurement device scheme.
**Figure 2:**   Working principle of sensor and sensor tip.
**Figure 3:**   Sample application of measurement device.
**Figure 4:**   Diagram of muscle response to electrical stimulus measured using this invention and simultaneously measured M-wave (EMG response signal to surface electrical stimulation) muscle response to the same electrical stimulus.
**Figure 5:**   Diagram of muscle signal measured using this invention during volatile muscle activity and simultaneously measured EMG signal.

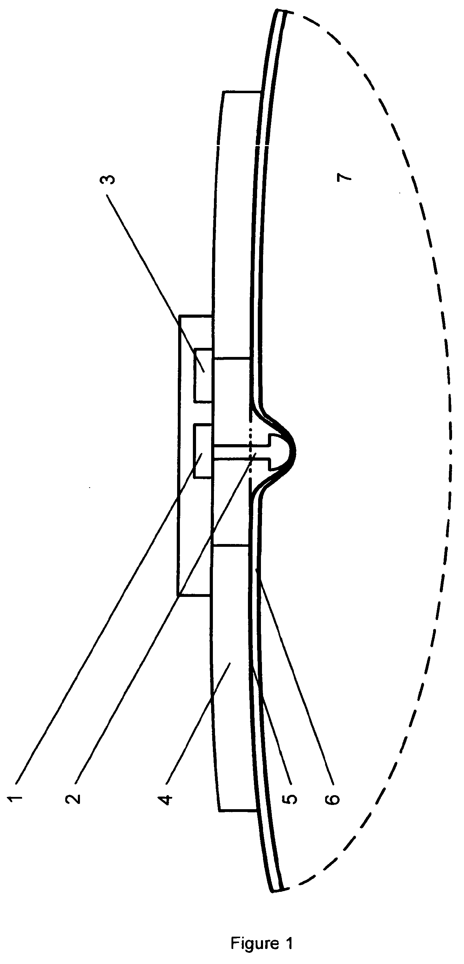

The measuring device (A) is presented in Figure 1. It consists of a sensor (1) with a sensor tip (2), microprocessor (3) and a supporting part (4). The latter binds all of the comprised measurement device parts together. All mentioned sensor parts are positioned on the skin surface (5) and through the intermediate layer (6) indirectly contacts the subject of measurement (7). As illustrated, for BCVP determination, the measuring device is pressed onto the surface of the individual's skin (5) above the muscle of interest (7). The measuring device (A) is constructed in such a way that its pressing on the individual's skin surface (5) above the subject of measurement (7) causes the device sensor (1) and sensor tip (2) to strain the surface of the measuring individual's skin (5) and the intermediate layer (6), ultimately putting pressure on the subject of measurement (7).

The depth to which the sensor tip (2) presses into the skin surface varies with the different physical characteristics of measuring individuals. For example, when estimating the BCVP of healthy individuals, the device sensor tip (2) pressed to a depth of a couple of millimetres. However, when performing measurements on an individual with a high percentage of body fat, the sensor tip (2) depth increases. If the initial sensor tip (2) position is not adequate, other tissue and fat surrounding the skeletal muscle will interfere with BCVP determination. The sensor tip (2) is shaped in such a way that the required depth of penetration is non-invasive and should not cause any pain or discomfort to the individual.

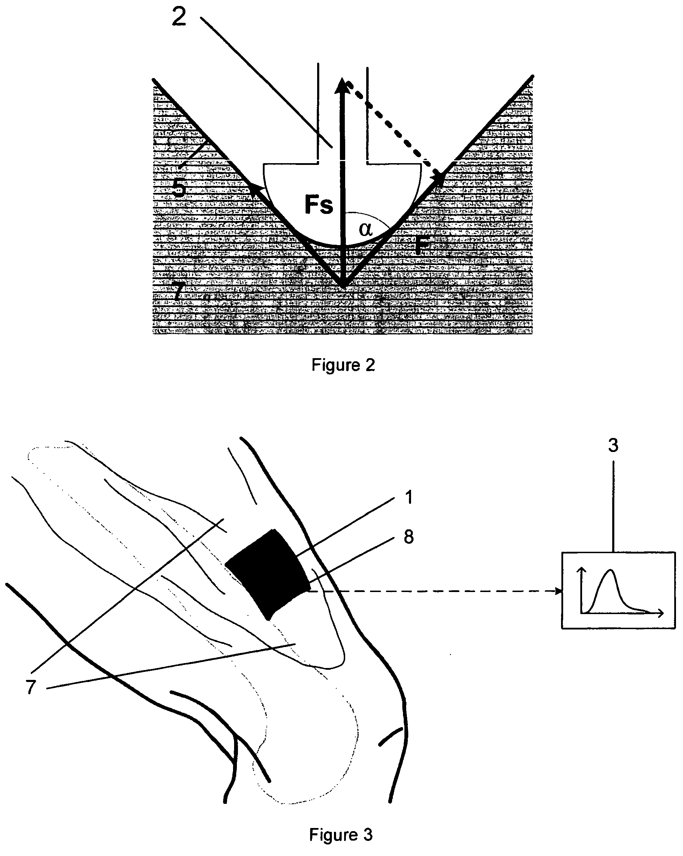

For efficient BCVP determination, the sensor tip (2) position relative to the subject of measurement (skeletal muscle, muscle part, tendon or ligament) must remain constant during the entire measurement procedure - the sensor tip (2) depth cannot change or incline in any direction. As illustrated in Figure 3, the supporting part (4) of the measuring device (A) and a special attachment (8), provide for a positioning that meets these requirements.

The device sensor (1) can include any suitable force or pressure meter without changing the tip position with the measured force. Such force meters may include meters that are based on the piezoelectronic effect, such as quartz piezoelectric force sensors and metal-foil strain-gauge meters that are used to convert the pressure force on the sensor tip (2) into an electrical signal in which the change in capacitance, inductance, or resistance of the electrical element in the sensor (1) is proportional to the strain experienced by the sensor (1).

Any other suitable force meter can be incorporated into the measurement device (A). A computer or microprocessor (3) is used to collect data and calculate and handle measurement signals.

Figure 2 illustrates the innovative measuring principle for the BCVP determination of skeletal muscle. The principle is characterised by a procedure that includes the positioning and fixation of the measuring sensor (1) on the skin surface (5) above the subject of measurement (7) and measuring the force acting on that sensor (1).

As illustrated in Figure 2, deepening a suitably shaped sensor tip (2) into the skin surface (2) above the subject of measurement (7) produces a force on the tip. During subject (7) activity, which is manifested by repeating contractions and retractions, the force acting on the sensor tip (2) changes. If the sensor tip (2) depth is suitable and does not change during measurement, the force change detected is entirely due to the subject of measurement (7) activity. Using adequate equipment, the force acting on the pressed sensor tip (2) can be measured to determine the BCVP of skeletal muscle or those of any other subject of measurement.

The resultant force that is measured with the device (A) is a vector sum of two forces present at the surface of the subject of measurement (7). The simplified resultant force can be expressed through the following equation:

$$Fs = 2 * F * \cos(\alpha).$$

Figure 3 shows a sample application of the measurement device (A). The measuring device (A) contacts the skin surface (5) above the skeletal muscle (7) using a special attachment (8). The optimal position of the measuring device (A) on the skin surface (5) depends on the subject to be measured. The measuring device (A) and method can be equally applied to determine the BCVP of a specific skeletal muscle part, tendon or ligament. In such cases, the measuring device (A) is positioned precisely above the subject of interest.

To enable efficient BCVP determination, the measuring device (A) must securely contact the skin surface (5) to ensure that the measurement obtained is due to skeletal muscle activity and not due to the movement of the sensor (1) and sensor tip (2). Furthermore, if the measurements are to be performed on moving individuals performing some activity, the measuring device (A) and its attachment must not limit the individual's movement. The position of the measuring device (A) provided by the attachment part (8) has to conform to the individual's body and must be secure, preventing shifting of the sensor tip (2) relative to the subject of measurement (7).

Attachment parts (8) that are in accordance with the abovementioned requirements can come in different shapes and sizes. Suitable attachments can be made of, but are not limited to, straps or adhesive plasters. Any other attachment that fulfils the abovementioned requirements can be used as well.

The measurement method can be applied to measure BCVP during volatile muscle activity (muscle activity that is under the individual's control) during electrically or magnetically stimulated muscle activity or any other different way in which muscle activity is provoked (change of viscoelastic and contractile properties).

When measuring volatile muscle activity, the measuring individual provokes activity from the muscle to which the measuring device is attached. Muscle activities manifested by repeated muscle contraction and retraction are measured according to the innovative measuring method.

BCVP can also be determined during muscle length changes (stretch shortening cycle). For example, when a measuring individual is contracting his or her knee muscles (quadriceps muscles), the tension in the muscles associated with the knee angle changes. This particular change in muscle tension can be determined by measuring the force according to the innovative measurement method and device (A) herein presented.

When measuring a muscle's response to electrical stimulation, additional means are used in the measuring procedure to stimulate the skeletal muscle. The measurement device can still be used as illustrated in previous examples, when determining skeletal muscle BCVP during volatile activity.

The obtained BCVP measurements results are illustrated in Figure 3, which were collected and processed by the device computer or microprocessor (3).

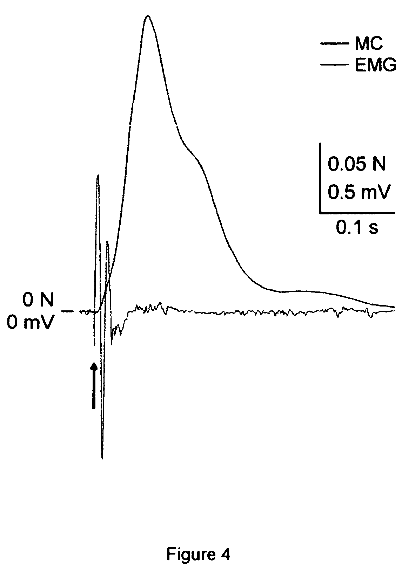

Figure 4 shows sample muscle response measured using the innovative measuring device and method. The presented measured signal is obtained by electrically stimulating muscle activity. For comparison purposes, the simultaneously measured M-wave muscle response to the same electrical stimulant is also presented.

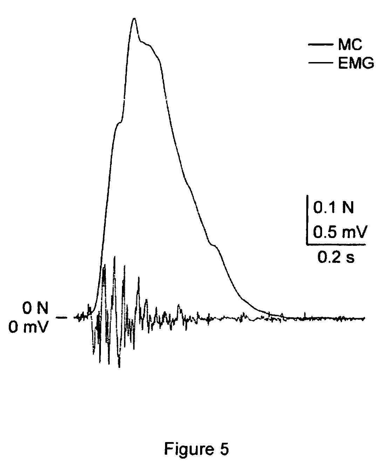

Figure 5 shows sample BCVP obtained during volatile muscle activity using the innovative measuring device and method. The measurement presented includes data measured during one muscle contraction and retraction cycle. The measured response is compared to the simultaneously measured EMG signal.

Until now, the methods used for measuring muscle force have represented a significant technical problem. No measuring method in use today offers a dominant advantage that would make it generally applicable.

The measuring method and device for the determination of the BCVP of skeletal muscle described in this document, offer a number of characteristic advantages over existing methods and devices, making them generally applicable and useful. Both the measuring method and device can be applied to perform non-invasive *in situ* BCVP determination of various subjects (skeletal muscles, muscle parts, tendons and ligaments). The attachment of the measuring device does not cause any pain or discomfort to the measuring individual. Furthermore, as the measurements are performed *in situ,* muscle BCVP determination can be localised to a specific skeletal muscle part of interest. The innovative measurement method and device provide for the use of the same equipment for the determination of the BCVP of different skeletal muscles. Accordingly, measurers can perform measurements more easily and faster than when using different measuring equipment for different skeletal muscles.

The method described can be applied to determine the BCVP of skeletal muscle during volatile muscle activity, muscle response to an electrical or magnetic stimulation or any other change in muscle activity (viscoelastic or contractile properties). Furthermore, the attachment of the measuring device and measurement performance does not limit the individual's movement or activity. The determination of the BCVP of skeletal muscle can therefore be performed equally and with same measuring equipment for still as well as moving individual.

Moreover, the measuring method allows for the determination of muscle activity changes during some activity or body movement that the measuring individual is performing. These changes include changes in muscle activation pattern, recruitment order, activity intension and other changes during the individual's activity or movement performance. Therefore, the measuring method and the device are applicable for skeletal muscle BCVP determination during different activities (running, jumping, etc.) and body movements, enabling a better understanding of muscle and muscle part BCVP analysis and better functional diagnostics.

The innovative measurement method and device can be used to determine the influence of different agents that can change normal muscular and skeletal system behaviours, such as different medications, stimulants, and substances that influence the activity of the central nervous system.

All of the described characteristics provide for a distinct advantage of the innovative measuring method and device over the methods that are in use today. The measurements obtained using this method and device are relevant and accurate and can benefit various fields, such as medicine and physiotherapy.

**Claims**

1. Measurement device (A) for selective and non-invasive determination of biomechanical, contractile and viscoelastic properties of a subject (7) of measurement from the group of skeletal muscles, muscle parts, tendons, and ligaments, the device (A) comprising: a force or pressure measurement sensor (1) with a sensor tip (2), which is adapted to be pressed into the skin surface (5) above the subject (7) of measurement, a microprocessor (3), a supporting part (4), which provides for the suitable positioning of the measurement device (A) on the skin surface (5), and an attachment means (8), wherein the supporting part (4) along with the attachment (8) provide for the attachment and fixation of the device (A) on the skin surface (5),
**characterized in that**
the supporting part (4) is adapted to contact the skin surface (7) and to be pressed onto the skin surface (5) by the attachment means (8), and
the attachment means is made of an adhesive plaster.

2. Measurement device (A) according to claim 1 **characterised in that** the measurement sensor (1) is a quartz piezoelectric force sensor or a metal-foil strain-gauge meter.

**Patentansprüche**

1. Messvorrichtung (A) zur selektiven und nichtinvasiven Bestimmung biomechanischer, kontraktiler und viskoelastischer Eigenschaften eines Messsubjekts (7) aus der Gruppe von Skelettmuskeln, Muskelteilen, Sehnen und Ligamenten, die Messvorrichtung umfassend: einen Kraft- oder Druckmesssensor (1) mit einer Sensorspitze (2), die zur Einpressung in die Hautoberfläche (5) oberhalb des Messsubjekts (7) angepasst ist, einen Mikroprozessor (3), ein Stützteil (4), welches die richtige Positionierung der Messvorrichtung (A) auf der Hautoberfläche (5) ermöglicht, sowie ein Befestigungsmittel (8), wobei das Stützteil (4) mit der Befestigung (8) eine Befestigung und Fixierung der Vorrichtung (A) auf der Hautoberfläche (5) ermöglicht,
**dadurch gekennzeichnet, dass**
das Stützteil (4) angepasst ist, die Hautoberfläche (7) zu berühren und (5) mit dem Befestigungsmittel (8) in die Hautoberfläche eingepresst zu werden, und das Befestigungsmittel aus einem Klebepflaster hergestellt ist.

2. Messvorrichtung (A) nach Anspruch 1
**dadurch gekennzeichnet, dass**
der Messsensor (1) ein Quarz-piezoelektrischer Kraftsensor oder ein Metallfolien-Dehnungsmessstreifen ist.

**Revendications**

1. Dispositif de mesure (A) destiné à déterminer de manière sélective et non invasive les propriétés biomécaniques, contractiles et viscoélastiques d'un sujet de mesure (7) du groupe de muscles, squelettiques, de parties musculaires, de tendons et de ligaments, le dispositif (A) comprenant : un capteur de mesure de force ou de pression (1) avec une pointe de capteur (2) ayant une forme adaptée à être pressée dans la surface de la peau (5) au-dessus du sujet de mesure (7), un microprocesseur (3), une partie de support (4) qui permet le bon positionnement du dispositif de mesure (A) à la surface de la peau (5), et un moyen de fixation (8), la partie de support (4) et la fixation (8) permettant la fixation du dispositif (A) à la surface de la peau (5),
**caractérisé en ce que**
la partie de support (4) est adaptée pour être en contact avec la surface de la peau (7) et pour être pressée dans la surface de la peau (5) par le moyen de fixation (8), et le moyen de fixation étant fabriqué d'un pansement adhésif.

2. Dispositif de mesure (A) selon la revendication 1
**caractérisé en ce que**
le capteur de mesure (1) est un capteur de force piézoélectrique quartz ou une jauge de déformation à feuille métallique.

Figure 1

Figure 2

Figure 3

Figure 4

Figure 5

**EP 2 590 557 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 102008038234 A1

### Non-patent literature cited in the description

- **S. NIELSEN ; B.K. PEDERSEN.** Skeletal muscle as an immunogenic organ. *Curr Opin Pharmacol,* June 2008, vol. 8 (3), 346-51
- **L.R. LEIBER.** Skeletal Muscle Structure, Function and Plasticity: The Physiological Basis of Rehabilitation. Lippincott Williams and Wilkins, 2002
- **B.K. PEDERSEN ; T.C.A. AKERSTRÖM ; A. R. NIELSEN ; C.P. FISCHER.** Role of myokines in exercise and metabolism. *Appl Physiol,* 10 September 2007, vol. 3 (3), 1093-8
- **B.K. PEDERSEN ; C.P. FISCHER.** Beneficial health effects of exercise--the role of IL-6 as a myokine. *Trends Pharmacol Sci.,* April 2007, vol. 28 (4), 152-6
- **R. KOOPMAN ; L.J.C. VAN LOON.** Aging, exercise, and muscle protein metabolism. *J Appl Physiol,* 10 June 2009, vol. 6 (6), 2040-8
- **G. PARISE ; S.M. PHILLIPS ; J.J. KACZOR ; M.A. TAMOPOLSKY.** Antioxidant enzyme activity is up-regulated after unilateral resistance exercise training in older adults. *Free Radic Biol Med.,* 15 July 2005, vol. 39 (2), 289-95
- **G. PARISE ; A.N. BROSE ; M.A. TARNOPOLSKY.** Resistance exercise training decreases oxidative damage to DNA and increases cytochrome oxidase activity in older adults. *Exp Gerontol.,* March 2005, vol. 40 (3), 173-80
- **B.K. HIGGINSON.** Methods of running gait analysis. *Curr Sports Med Rep,* May 2009, vol. 8 (3), 136-41
- **H. CLARKSON ; G. GILEWICH.** Musculoskeletal assessment: joint range of motion and manual muscle strength. Williams and Wilkins, 1989
- **T. FUKUNAGA ; R.R. ROY ; F.G. SHELLOCK ; J.A. HODGSON ; M.K. DAY ; P.L. LEE ; H. KWONG-FU ; V.R. EDGERTON.** Physiological cross-sectional area of human leg muscles based on magnetic resonance imaging. *J Orthop Res,* November 1992, vol. 10 (6), 928-34
- Neuromuscular basis of the maximum voluntary force capacity of muscle. **R.M. ENOKA ; A.J. FUGLEVAND.** Current Issues in Biomechanics. 1993, 215-235

- **M. ITO ; H. AKIMA ; T. FUKUNAGA.** In vivo moment arm determination using B-mode ultrasonography. *J Biomech,* February 2000, vol. 33 (2), 215-8
- **Y. ICHINOSE ; Y. KAWAKAMI ; M. ITO ; H. KANEHISA ; T. FUKUNAGA.** In vivo estimation of contraction velocity of human vastus lateralis muscle during ''isokinetic'' action. *J Appl Physiol,* March 2000, vol. 88 (3), 851-6
- **R.L. LIEBER.** Isokinetic Dynamometers Used in Physical Assessment, Skeletal Muscle Structure, Function, and Plasticity. Lipincott Williams & Wilkins, 2010, 119-124
- **L. GERILOVSKY ; P. TSVETINOV ; G. TRENKOVA.** Peripheral effects on .the amplitude of monopolar and bipolar H-reflex potentials from the soleus muscle. *Exp Brain Res,* 1989, vol. 76 (1), 173-81
- **B. BIGLAND-RITCHIE.** EMG/force relations and fatigue of human voluntary contractions. *Exerc Sport Sci Rev,* 1981, vol. 9, 75-117
- **A.L. HOF ; J. VAN DEN BERG.** EMG to force processing I: An electrical analogue of the Hill muscle model. *J Biomech,* 1981, vol. 14 (11), 747-58
- **A.L. HOF ; J. VAN DEN BERG.** EMG to force processing II: Estimation of parameters of the Hill muscle model for the human triceps surae by means of a calfergometer. *J Biomech,* 1981, vol. 14 (11), 759-70
- **A.L. HOF ; J. VAN DEN BERG.** EMG to force processing III: Estimation of model parameters for the human triceps surae muscle and assessment of the accuracy by means of a torque plate. *J Biomech,* 1981, vol. 14 (11), 771-85
- **A.L. HOF ; J. VAN DEN BERG.** EMG to force processing IV: Eccentric-concentric contractions on a spring-flywheel set up. *J Biomech,* 1981, vol. 14 (11), 787-92
- **H.S. MILNER-BROWN ; R.B. STEIN.** The relation between the surface electromyogram and muscular force. *J Physiol,* April 1975, vol. 246 (3), 549-69
- **R.M. ENOKA.** *Electromyography, Neuromechanics of human movement, Champaign, USA: Human Kinefics,* 2002, 46.55

- **T.W. BECK ; T.J. HOUSH ; J.T. CRAMER et al.** Mechanomyographic amplitude and frequency responses during dynamic muscle actions: a comprehensive review. *BioMed Eng OnLine,* 2005, vol. 4 (1), 67
- **T. W. BECK ; T J. HOUSH ; G.O. JOHNSON et al.** Mechanomyographic and electromyographic amplitude and frequency responses during fatiguing isokinetic muscle actions of the biceps brachii. *Electromyogr Clin Neurophysiol,* 2004, vol. 44 (7), 431-444
- **C. ORIZIO.** Sound myogram and EMG cross-spectrum during exhausting isometric contractions in humans. *J Electromyogr Kinesiol,* 1992, vol. 2 (3), 141-149
- **C. ORIZIO.** Muscle sound: bases for the introduction of a mechanomyographic signal in muscle studies. *Crit Rev Biomed Eng,* 1993, vol. 21 (3), 201-243

- **G. GORDON ; A.H.S. HOLBOUM.** The sounds from single motor units in a contracting muscle. *J Physiol,* 1948, vol. 107 (4), 456-464
- **T.W. BECK ; T.J. HOUSH ; J.T. CRAMER ; J. P. WEIR ; G.O. JOHNSON ; J.W. COBUM ; M.H. MALEK ; M. MIELKE.** Mechanomyographic amplitude and frequency responses during dynamic muscle actions: a comprehensive review. *Biomed Eng Online,* 19 December 2005, vol. 4 (1), 67
- **R. DAHMANE ; V. VALENČIČ ; N.KNEZ ; I. ERŽEN.** Evaluation of the ability to make non-invasive estimation of muscle contractile properties on the basis of the muscle belly response. *Medical and Biological Engineering and Computing,* 2006, vol. 39 (1), 51-55